Europäisches Patentamt

European Patent Office

Office européen des brevets

(11) Publication number: **0 118 534**
**B1**

## EUROPEAN PATENT SPECIFICATION

(45) Date of publication of patent specification: **08.04.87**

(21) Application number: **83902968.3**

(22) Date of filing: **25.08.83**

(86) International application number:
**PCT/US83/01299**

(87) International publication number:
**WO 84/00978 15.03.84 Gazette 84/07**

(51) Int. Cl.⁴: **C 12 Q 1/48**

(54) METHOD FOR DETERMINING -g(g)-GLUTAMYLTRANSFERASE ACTIVITY AND KITS CONTAINING A NOVEL SUBSTRATE SOLUTION FOR USE THEREIN.

(30) Priority: **30.08.82 US 413042**

(43) Date of publication of application:
**19.09.84 Bulletin 84/38**

(45) Publication of the grant of the patent:
**08.04.87 Bulletin 87/15**

(84) Designated Contracting States:
**CH DE FR GB LI NL SE**

(56) References cited:
**EP-A-0 006 582**
**EP-A-0 018 628**
**FR-A-2 106 163**
**US-A-3 892 631**
**US-A-3 986 931**
**US-A-4 310 624**
**US-A-4 310 625**

(73) Proprietor: **BECKMAN INSTRUMENTS, INC.**
**Executive Office 2500 Harbor Boulevard**
**Box 3100**
**Fullerton California 92634 (US)**

(72) Inventor: **BECK, James, P.**
**314 Prospect Street**
**Newport Beach, CA 92663 (US)**
Inventor: **LUNA, Carlos, E.**
**2322 Cayuga Avenue**
**Placentia, CA 92670 (US)**

(74) Representative: **Arthur, John William et al**
**FITZPATRICKS 4 West Regent Street**
**Glasgow G2 1RS Scotland (GB)**

(56) References cited:
**Chemical Abstracts, volume 95, no. 1, 6 July 1981, Columbus, Ohio (US). F. Schiele et al.: "Measurement of plasma gama-glutemyltransferase in clinical chemistry: kinetic basis and standardization propositions", see page 246, column 2, abstract no. 2354d, Clin.Chim.Acta 1981, 187-195 (Eng) Clinical Chemistry, volume 27, no. 2, February 1981, Easton, Pennsylvania, (US). H. E. Solberg et al.: "8-Glutamyltransferase in Human Serum: An analysis of Kinetic Models", see pages 303-307**

Courier Press, Leamington Spa, England.

## 0 118 534

**Description**

BACKGROUND OF THE INVENTION

1. Field of the Invention

This invention relates to a method for determining γ-glutamyltransferase activity as well as to a kit containing a novel substrate solution for use therein.

2. Description of the Prior Art

γ-glutamyltransferase [(γ-glutamyl)-peptide:-amino acid γ-glutamyltransferase (EC 2.3.2.2)], discovered in 1950 by Hanes et al. (1, 2), catalyzes the transfer of the γ-glutamyl group from a γ-glutamyl peptide to an amino acid or another peptide.

Although γ-glutamyltransferase is widely distributed in human organs, an increase in its activity in serum is almost specifically an indicator of diseases of the hepatobiliary track or of hepatic involvement in the primary elements (3—7).

For determination of γ-glutamyltransferase activity, L-γ-glutamyl-p-nitroanilide (8) is at present almost invariably used as a substrate, because it allows a direct reaction rate measurement without deproteinization or any chemical treatment of the cleavage product, p-nitroaniline. Recently, a more soluble derivative of L-γ-glutamyl-p-nitroanilide, namely, L-γ-glutamyl-3-carboxy-p-nitroanilide has been described as an alternative substrate in the γ-glutamyltransferase assay (9).

Prior art solutions of both L-γ-glutamyl-3-carboxy-p-nitroanilide and L-γ-glutamyl-p-nitroanilide suffer from stability and solubility problems in their presently employed aqueous environments. For example, the relatively short shelf-life of aqueous solutions of both L-γ-glutamyl-3-carboxy-p-nitroanilide and L-γ-glutamyl-p-nitroanilide have necessitated the freeze-drying thereof to prolong the shelf-life of these products. To reconstitute the freeze-dried product requires one to place it in water and heat the resulting mixture to up to about 50°C. This reconstitution step is time consuming, causes greater variations in the resulting reagent, as well as a loss of substrate. In addition, the freeze-drying procedure itself creates larger lot-to-lot variations.

Accordingly, it would be very advantageous to develop a novel substrate solution for use in the assay of γ-glutamyltransferase wherein such substrate solution comprises a substrate dissolved in a matrix such that the resulting substrate solution possesses increased stability and overcomes the solubility problems present in prior art.

Summary of the Invention

In accordance with the present invention, there is provided a substantially anhydrous substrate solution (hereinafter referred to as novel substrate solution) comprising (a) a substrate selected from salts of L-γ-glutamyl-p-nitroanilide and (b) a polyol selected from alkyl polyols containing 2 to 10 carbon atoms and 2 to 10 hydroxy groups and polyethylene glycol having an average molecular weight of from 200 to 600.

In addition, the instant invention provides a kit for the determination of γ-glutamyltransferase activity. The kit of the instant invention is of the type comprising (a) a buffered medium comprising a buffer and an acceptor for γ-glutamyl residue; and (b) a substrate solution. The buffered medium and substrate solution are adapted such that preselected aliquots thereof can react in the presence of γ-glutamyltransferase to produce an absorbing cleavage product; characterized in that the above described novel substrate solution is employed therein.

Furthermore, the instant invention also encompasses a method for determining γ-glutamyltransferase activity. The method of the instant invention is of the type comprising (a) contacting in any preselected order an aliquot of a buffered medium comprising a buffer and a γ-glutamyl residue acceptor, an aliquot of a substrate solution, and an aliquot of a sample to be assayed to thereby form a cleavage product and (b) measuring the absorbance of the cleavage product; characterized in that the above-described novel substrate solution is employed therein.

Description of the Preferred Embodiments

The novel substrate solution of the instant invention comprises a substrate and a polyol. Virtually any salt of L-γ-glutamyl-p-nitroanilide and L-γ-glutamyl-3-carboxy-4-nitroanilide can be employed as the substrate in the instant invention. Preferably, the substrate is selected from L-γ-glutamyl-p-nitroanilide hydrohalide and L-γ-glutamyl-3-carboxy-4-nitroanilide monoammonium salts. More preferably, the substrate is selected from the hydrochloride, hydrobromide, and hydroiodide salts of L-γ-glutamyl-p-nitroanilide. L-γ-glutamyl-p-nitroanilide hydrochloride salt is the substrate of choice.

The substrate solution of the present invention can comprise from 25 to 250 mmol/l substrate. Preferably, the composition of the instant invention comprises from 40 to 100 mmol/l substrate. Optimally, the composition comprises 50 mmol/l substrate.

The polyol employed in the substrate solution of the instant invention is selected from alkyl polyols containing from 2 to 10 carbon atoms and from 2 to 10 hydroxy groups and polyethylene glycol having an average molecular weight of from 200 to 600. Preferably, the polyol is selected from alkyl polyols containing from 2 to 5 carbon atoms and from 2 to 4 hydroxyl groups and polyethylene glycol having an

2

average molecular weight of from 200 to 400. More preferably, the polyol is selected from glycerol, ethylene glycol, propylene glycol, 1,3-propanediol, butylene glycol, pentanediol, polyethylene glycol 200, and polyethylene glycol 300. Ethylene glycol, propylene glycol, polyethylene glycol 200, and polyethylene glycol 300 are the polyols of choice for use in the substrate solution of the instant invention.

The substrate solution of the instant invention is preferably an anhydrous system. However, a water content of no greater than 10%, preferably no greater than 5%, v/v is acceptable. Accordingly, the substrate solution of the instant invention optionally further comprises up to 20% v/v of an inert hydroscopic solid. More preferably, the substrate solution of the instant invention further comprises up to 5% v/v of the inert hydroscopic solid. The function of the optional inert hydroscopic solid is to achieve the preferred anhydrous embodiment of the substrate solution of the instant invention. The hydroscopic solid must be inert, an efficient water absorber, and of neutral or alkaline pH. The solid is preferably a high area hydroscopic agent such as a natural or synthetic molecular sieve having a particle size of from about 2 to about 16 mesh. The amount of surface area is important since the material acts to absorb water into the pores.

Molecular sieves are zeolites whose atoms are arranged in crystal lattice in such a way that there is a large number of small cavities interconnected by smaller openings or pores of precisely uniform size. Normally, these cavities contain water molecules, but upon heating under vacuum, this water is driven off without any change in the remaining crystal lattice. The network of cavities and pores may occupy 50% of the total volume of the crystals. Molecular sieves have a strong tendency to reabsorb water, and other small molecular weight liquids.

A few natural zeolites exhibit molecular sieve characteristics to a limited degree. Synthetic zeolites are available in several sizes (pore openings 3, 4 and 10 Angstrom units (0.3, 0.4 and 1 nm) in diameter) with high capacity for absorption and regeneration even when used at elevated temperatures.

In general, the substrate solution of the instant invention can be made by dissolving a hydrohalide salt of L-γ-glutamyl-p-nitroanilide in a polyol, preferably an anhydrous polyol, at room temperature.

An alternate method to prepare the substrate solution is to add L-γ-glutamyl-p-nitroanilide free base to the polyol followed by the addition of a hydrohalide acid (in aqueous solution) to form a hydrohalide salt in situ. If one desires an anhydrous substrate solution, this method would require the addition of a molecular sieve to remove the added water.

The preferred technique for making the substrate solution of this invention entails the additon of L-γ-glutamyl-p-nitroanilide hydrochloride to anhydrous propylene glycol at room temperature with constant mixing to yield the desired solution.

Buffers which can be employed in the buffering medium of the kit of this invention include, but are not limited to, tris(hydroxymethyl)amino methane and any other buffer capable of maintaining the desired pH, preferably pH 8.1 ± 0.2, and which does not interfere in the recovery of γ-glutamyltransferase activity. The preferred buffer is tris(hydroxymethyl)amino methane.

Suitable γ-glutamyl residue acceptors include, but are not limited to, glycyglycine and S-methyl-L-cysteine. The preferred γ-glutamyl residue acceptor is glycyglycine.

A working reagent can be prepared by mixing an aliquot of the substrate solution with an aliquot of the buffered medium. Preferably, the working reagent is prepared by adding one part of the substrate solution to ten parts of the buffered medium and mixing the resulting solution.

The substrate solution and buffered medium should be formulated such that after mixing an aliquot of the substrate solution with an aliquot of the buffered medium in a ratio of from 1:5 to 1:50 to form the working reagent, the concentration of substrate in the working reagent is from 1 to 10 mmol/L.

Although the sample to total volume ratio can vary, a 1:21 ratio has been found very satisfactory.

The following examples are provided for the purpose of further illustration only and are not intended to be limitations on the disclosed invention.

## Example 1
A preferred γ-glutamyltransferase reagent kit has the following formulation:

Constituents

| Buffered Medium | Preferred | More Preferred | Optimal |
|---|---|---|---|
| Tris(hydroxymethyl)aminomethane, m molar | 90—110 | 95—105 | 100 |
| Glycylglycine, m molar | 150—180 | 160—170 | 165 |
| Magnesium chloride, hexahydrate, m molar | 79—97 | 83—93 | 88 |
| Sodium azide | 0.8%—1.2% | 0.9%—1.1% | 1% |
| Sodium hydroxide as needed to adjust pH to: | 8.1±0.2 | 8.1±0.5 | 8.1±0.1 |
| Purified water | Q.S. to make desired volume | | |

Substrate Solution

Propylene glycol, anhydrous Q.S. to make desired volume

| | | | |
|---|---|---|---|
| γ-L-glutamyl-p-nitroanilide hydrochloride, m molar | 45—55 | 47—53 | 50 |

*Q.S. denotes a sufficient quantity.

## Example 2
Various control and patient sera samples were assayed on a centrifugal fast analyzer in a correlation study employing the optimal γ-glutamyltransferase reagent kit of Example 1 and a commercially available γ-glutamyltransferase reagent kit and the results obtained therefrom are set forth in Table I.

TABLE I

| Sample | Commercial Kit (X) | γ-Glutamyl-transferase Kit Within Scope of Invention (Y) |
|---|---|---|
| | IU/L | |
| Decision™ 1 | 20 | 17 |
| Decision™ 2 | 55 | 51 |
| Decision™ 3 | 114 | 108 |
| Linearity Control 1 | 17 | 14 |
| Linearity Control 2 | 35 | 32 |
| Linearity Control 3 | 64 | 62 |
| Linearity Control 4 | 123 | 120 |
| Linearity Control 5 | 240 | 238 |
| Linearity Control 6 | 357 | 354 |
| Linearity Control 7 | 485 | 480 |
| Linearity Control 8 | 592 | 589 |
| Patient Serum 1 | 9 | 8 |
| Patient Serum 2 | 34 | 31 |
| Patient Serum 3 | 21 | 20 |
| Patient Serum 4 | 26 | 25 |
| Patient Serum 5 | 21 | 20 |
| Patient Serum 6 | 25 | 23 |
| Patient Serum 7 | 10 | 10 |
| Patient Serum 8 | 26 | 27 |
| Patient Serum 9 | 57 | 58 |
| Patient Serum 10 | 81 | 84 |
| Patient Serum 11 | 19 | 21 |
| Patient Serum 12 | 24 | 24 |
| Patient Serum 13 | 22 | 26 |

The data set forth in Table I gives a correlation of $Y = 0.993X - 0.5 IU/L$ and a correlation coefficient (r) of 0.999. Accordingly, this data indicates that the results obtained via a γ-glutamyltransferase reagent within the scope of this invention correlate well with those obtained with a commercially available kit.

## Example 3

Stability Study

A fresh reagent within the scope of this invention and having a formulation as set forth in the "optimal" column of Example 1 was used to value assign a lot of Decision™ controls and an in-house series of linearity control materials. These value assignments are set forth in Table II.

The fresh reagent was then packaged and incubated at 41°C for 2 days in order to approximate one year storage at 5°C.

After the second day at 41°C, the reagent was then divided into two sets. One set was then stored at an incubation temperature of 30°C and the other set was then stored at an incubation temperature of 37°C. Aliquots from both sets were periodically taken and employed in an γ-glutamyltransferase assay. The results obtained are also set forth in Table II.

TABLE II

| Sample | | Value Assigned±S.D.* | After 2 days @ 41°C | day at 30°C | | | day at 37°C | | |
|---|---|---|---|---|---|---|---|---|---|
| | | | | 1 | 2 | 3 | 1 | 2 | 3 |
| Decision | 1 | 19.7 ± 1.8 | 21.9 | 19.1 | 20.0 | 21.7 | 19.6 | 23.9 | 20.0 |
| Decision | 2 | 53.5 ± 1.9 | 55.0 | 52.5 | 53.8 | 55.6 | 53.7 | 52.9 | 50.7 |
| Decision | 3 | 111 ± 1.8 | 113 | 111 | 111 | 111 | 110 | 106 | 104 |
| Linearity Control | 1 | 15.7 ± 1.8 | 19.0 | 16.8 | 17.4 | 19.3 | 18.9 | 21.9 | 16.6 |
| Linearity Control | 2 | 34.3 ± 2.1 | 37.5 | 35.3 | 36.4 | 37.1 | 36.1 | 38.6 | 30.0 |
| Linearity Control | 3 | 64.5 ± 1.6 | 67.0 | 67.0 | 65.0 | 67.0 | 67.0 | 64.0 | 61.4 |
| Linearity Control | 4 | 125 ± 3.6 | 126 | 125 | 122 | 125 | 122 | 121 | 116 |
| Linearity Control | 5 | 245 ± 3.5 | 233 | 244 | 239 | 242 | 242 | 238 | 235 |
| Linearity Control | 6 | 362 ± 3.5 | 361 | 361 | 351 | 356 | 355 | 354 | 339 |
| Linearity Control | 7 | 488 ± 3.5 | 479 | 477 | 468 | 479 | 477 | 475 | 463 |
| Linearity Control | 8 | 597 ± 4.1 | 582 | 587 | 583 | 581 | 583 | 582 | 570 |
| Absorbance @ 405 nm | | 0.549 | 0.566 | 0.595 | 0.583 | 0.615 | 0.591 | 0.626 | 0.493 |

*S.D. denotes standard deviation

The data set forth in Table II indicates the good stability of the γ-glutamyltransferase reagents of the instant invention.

Bibliography
1. Hanes et al., *Nature, 166*: 288—299 (1950).
2. Hanes et al., *Biochem, J., 51*: 25—35 (1952).
3. Kokot et al., *Z. Gesamte Inn. Med. Ihre Grenzgeb, 18*: 851—856 (1963).
4. Rutenburg et al., *Gastroenterology, 45*: 43—48 (1963).
5. Zein, *Lancet, ii*: 748—750 (1970).
6. Mayr, *Wien, Klin. Wochenschr., 85*: 83—87 (1973).
7. Schmidt et al., *Dtsch. Med. Wochenschr., 98*: 1572—1578 (1973).
8. Orlowski et al., *Biochim. Biophys. Acta, 73*: 679—681 (1963).
9. Persijn et al., *LAB., 3*: 108 (1976). Abstract.

## Claims

1. A substantially anhydrous substrate solution comprising:

(a) a substrate selected from salts of L-γ-glutamyl-p-nitroanilide; and

(b) a polyol selected from alkyl polyols containing 2 to 10 carbon atoms and 2 to 10 hydroxy groups and polyethylene glycol having an average molecular weight of from 200 to 600.

2. The substrate solution of claim 1 wherein:

(a) said substrate is selected from the hydrohalide salts of L-γ-glutamyl-p-nitroanilide; and

(b) said polyol is selected from alkyl polyols containing from 2 to 5 carbon atoms and from 2 to 4 hydroxyl groups and polyethylene glycol having an average molecular weight of from 200 to 400.

3. The substrate solution of claim 1 wherein:

(a) said substrate is selected from L-γ-glutamyl-p-nitroanilide hydrochloride, L-γ-glutamyl-p-nitroanilide hydrobromide, L-γ-glutamyl-p-nitroanilide hydroiodide salts; and

(b) said polyol is selected from glycerol, ethylene glycol, propylene glycol, 1,3-propanediol, butylene glycol, pentanediol, polyethylene glycol 200, and polyethylene glycol 300.

4. The substrate solution of claim 1 wherein:

(a) said substrate is L-γ-glutamyl-p-nitroanilide hydrochloride salt; and

(b) said polyol is selected from ethylene glycol, propylene glycol, polyethylene glycol 200, and polyethylene glycol 300.

5. The substrate solution of claim 4 comprising from 25 to 250 mmol/litre substrate.

6. The substrate solution of claim 4 comprising from 40 to 100 mmol/litre substrate.

7. The substrate solution of claim 4 comprising 50 mmol/litre substrate.

8. A kit for the determination of γ-glutamyltransferase activity of the type comprising:

(a) a buffered medium comprising a buffer and a γ-glutamyl residue acceptor; and

(b) a substrate solution;

said buffered medium and substrate solution being adapted such that preselected aliquots thereof can react in the presence of γ-glutamyltransferase to produce an absorbing cleavage product, characterized in that said substrate solution is the substantially anhydrous substrate claimed in any one of the preceding claims.

9. A method for determining γ-glutamyltransferase activity of the type comprising:

(a) contacting in any preselected order an aliquot of a buffered medium comprising a buffer and a γ-glutamyl residue acceptor, an aliquot of a substrate solution, and an aliquot of a sample to be assayed to thereby form a cleavage product; and

(b) measuring the absorbance of said cleavage product;

characterized in that said substrate solution is the substantially anhydrous substrate claimed in any of claims 1 to 7.

## Patentansprüche

1. Im wesentlichen wasserfreie Substratlösung umfassend

(a) ein aus Salzen von L-γ-Glutamyl-p-Nitroanilid ausgewähltes Substrat sowie

(b) ein aus 2 bis 10 Kohlenstoffatome und 2 bis 10 Hydroxyl-Gruppen enthaltenden Alkylpolyolen und Polyäthylenglykol mit einem durchschnittlichen Molekulargewicht von 200 bis 600 ausgewähltes Polyol.

2. Substratlösung nach Anspruch 1, in welcher

(a) das genannte Substrat aus den Hydrohalogenidsalzen von L-γ-Glutamyl-p-Nitroanilid gewählt ist und

(b) das gennannte Polyol aus 2 bis 5 Kohlenstoffatomen und 2 bis 4 Hydroxyl-Gruppen enthaltenden Alkylpolyolen und Polyäthylenglykol mit einem durchschnittlichen Molekulargewicht von 200 bis 400 gewählt ist.

3. Substratlösung nach Anspruch 1, in welcher

(a) das genannte Substrat aus Salzen von L-γ-Glutamyl-p-Nitroanilidhydrochlorid, L-γ-Glutamyl-p-Nitroanilidhydrobromid, L-γ-Glutamyl-p-Nitroanilidhydrojodid gewählt ist, und

(b) das genannt Polyol aus Glycerol, Äthylenglykol, Proplylenglykol, 1,3-Propandiol, Butylenglykol, Pentandiol, Polyäthylenglykol 200 und Polyäthylenglykol 300 gewählt ist.

4. Substratlösung nach Anspruch 1, in welcher

(a) das genannte Substrat L-γ-Glutamyl-p-Nitroanilidhydrochlorid-Salz ist und

(b) das genannte Polyol aus Äthylenglykol, Propylenglykol, Polyäthylenglykol 200 und Polyäthylenglykol 300 gewählt ist.

5. Substratlösung nach Anspruch 4, mit einem Gehalt von 25 bis 250 mmol/l Substrat.

6. Substratlösung nach Anspruch 4 mit einem Gehalt von 40 bis 100 mmol/l Substrat.

7. Substratlösung nach Anspruch 4, mit einem Gehalt von 50 mmol/l Substrat.

8. Set zur Bestimmung von γ-Glutamyltransferase- Aktivität vom Typ umfassend:

(a) ein einen Puffer und einen γ-Glutamyl-Rest-Akzeptor umfassendes gepuffertes Medium sowie

(b) eine Substratlösung,

wobei das gepufferte Medium und die Substratlösung so ausgebildet sind, daß vorgewählte Teilmengen hiervon in Gegenwart von γ-Glutamyltransferase unter Bildung eines absorbierenden Spaltungsproduktes reagieren können, dadurch gekennzeichnet, daß die Substratlösung das in einem der vorhergenden Ansprüchen beanspruchte im wesentlichen wasserfreie Substrat ist.

9. Verfahren zur Bestimmung von γ-Glutamyltransferase-Aktivität vom Typ umfassend die Schritte:

(a) Kontaktieren in einer beliebigen vorgegebenen Aufeinanderfolge einer Teilmenge eines einen Puffer und einen γ-Glutamyl-Rest-Akzeptor enthaltenden gepufferten Mediums, einer Teilmenge einer Substratlösung, sowie einer Teilmenge einer zu untersuchenden Probe, zur Bildung eines Spaltungsprodukts, und

(b) Messen des Absorptionsvermögens des gebildeten Spaltprodukts,

dadurch gekennzeichnet, daß die Substratlösung das in einem der Ansprüche 1 bis 7 beanspruchte im wesentlichen wasserfreie Substrat ist.

**Revendications**

1. Solution de substrat sensiblement anhydre comprenant:

(a) un substrat choisi parmi des sels de L-γ-glutamyl-p-nitroanilide; et

(b) un polyol choisi parmi des alkyl polyols contenant 2 à 10 atomes de carbone et 2 à 10 groupes hydroxy et du polyéthylène glycol ayant un poids moléculaire moyen de 200 à 600.

2. Solution du substrat de la revendication 1 où:

(a) ledit substrat est choisi parmi des sels d'hydrohalogénure de L-γ-glutamyl-p-nitroanilide; et

(b) ledit polyol est choisi parmi des alkyl polyols contenant de 2 à 5 atomes de carbone et de 2 à 4 groupes hydroxyles et du polyéthylène glycol ayant un poids moléculaire moyen de 200 à 400.

3. Solution du substrat de la revendication 1 où:

(a) ledit substrat est choisi parmi les sels de chlorhydrate de L-γ-glutamyl-p-nitroanilide, de bromhydrate de L-γ-glutamyl-p-nitroanilide, d'iodhydrate de L-γ-glutamyl-p-nitroanilide; et

(b) ledit polyol est choisi parmi le glycérol, l'éthylène glycol, le propylène glycol, le 1,3-propanediol, le butylène glycol, le pentanediol, le polyéthylène glycol 200 et le polyéthylène glycol 300.

4. Solution du substrat de la revendication 1 où:

(a) ledit substrat est le sel de chlorhydrate de L-γ-glutamyl-p-nitroanilide; et

(b) ledit polyol est choisi parmi l'éthylène glycol, le propylène glycol, le polyéthylène glycol 200 et le polyéthylène glycol 300.

5. Solution du substrat de la revendication 4 comprenant de 25 à 250 mmoles/litre du substrat.

6. Solution du substrat de la revendication 4 comprenant de 40 à 100 mmoles/litre du substrat.

7. Solution du substrat de la revendication 4 comprenant 50 mmoles/litre du substrat.

8. Ensemble pour la détermination de l'activité de la γ-glutamyltransférase du type comprenant:

(a) un milieu tamponné comprenant un tampon et un accepteur de résidu de γ-glutamyle; et

(b) une solution du substrat;

ledit milieu tamponné et ladite solution du substrat étant adaptés à ce que leurs aliquotes présélectionnées puissent réagir en présence de γ-glutamyltransferase pour produire un produit de scission absorbant, caractérisé en ce que ladite solution du substrat est le substrat sensiblement anhydre revendiqué selon l'une quelconque des revendications précédentes.

9. Procédé pour déterminer l'activité de la γ-glutamyltransférase du type consistant à:

(a) mettre en contact, en tout ordre présélectionné, une aliquote d'un milieu tamponné comprenant un tampon et un accepteur du résidu de γ-glutamyle, une aliquote d'une solution du substrat et une aliquote d'un échantillon à déterminer pour ainsi former un produit de scission; et

(b) mesurer l'absorbance dudit produit de scission;

caractérisé en ce que ladit solution du substrat est le substrat sensiblement anhydre revendiqué à l'une quelconque des revendications 1 à 7.